# EUROPÄISCHE PATENTSCHRIFT

(11) **EP 1 457 191 B1**
(45) Veröffentlichungstag und Bekanntmachung des Hinweises auf die Patenterteilung: **25.02.2009**
(21) Anmeldenummer: 04090068.0
(22) Anmeldetag: 25.02.2004
(51) Int. Cl.: A61K 8/25, A61Q 1/12

(54) **Kosmetischer Puder auf Siliciumdioxid-Basis und Herstellungsverfahren**
Cosmetic powder based on silicium dioxide and process for preparing it
Poudre cosmétique à base de dioxyde de silicium et son procédé de préparation

(30) Priorität: 13.03.2003 DE 10312124
(43) Veröffentlichungstag der Anmeldung: 15.09.2004
(73) Patentinhaber: Coty B.V., 2031 CC Haarlem (NL)
(72) Erfinder: Bleuez, Loïc, 06700 Saint Laurent du Var (FR); Golz-Berner, Karin, 98000 Monaco (MC); Porcu, Maryse, 06240 Beuasoleil (FR)
(74) Vertreter: Ziebig, Marlene

(56) Entgegenhaltungen:
- WO-A-01/37800
- WO-A-02/056844
- HASENZAHL, S., BRAUNAGEL, A.: "Fumed silica for personal care and cosmetics - versatile and effective" SÖFW JOURNAL SONDERDRUCK, Bd. 129, August 2003 (2003-08), Seiten 2-8, XP002289365 Gefunden im Internet: URL:http://www2.sivento.de/sivento/uploads _all/text/Fumed_silica_for_personal_care_a nd_cosmetics_-_versatile_and_effective.pdf > [gefunden am 2004-07-21]
- ""Trockenes Wasser" für die Haut" PRESSE-INFORMATION NR.1, [Online] 15. April 2004 (2004-04-15), XP002289366 DEGUSSA HOMEPAGE Gefunden im Internet: URL:https://www1.sivento.com/wps3/portal/a ction/PassContent/.reqid/-1?cqpath=http:// www2.sivento.de/sivento/uploads_all/text/A quaFoundation_en.pdf> [gefunden am 2004-07-20]
- "AEROSIL präsentiert die neusten Formulierungen auf Basis von "Trockenes Wasser""[Online] Mai 2004 (2004-05), XP002289367 DEGUSSA HOMEPAGE Gefunden im Internet: URL:https://www1.sivento.com/wps3/portal/e n/aerosil/more0?cqpath=http://149.216.91.4 9:4103/en/aerosil/more0&changeLang=de> [gefunden am 2004-07-20]
- DATABASE CHEMABS [Online] CHEMICAL ABSTRACTS SERVICE, COLUMBUS, OHIO, US; OKA, TAKASHI ET AL: "Cosmetic powders liquefying on application" XP002289379 gefunden im STN Database accession no. 2000:782691 & JP 2000 309505 A2 (SHISEIDO CO., LTD., JAPAN) 7. November 2000 (2000-11-07)

## Beschreibung

Die Erfindung betrifft einen Puder auf Siliciumdioxid-Basis, der eine deutlich verbesserte UV-Stabilität hat sowie dessen Herstellungsverfahren.

Es sind bereits eine Vielzahl von Pudern bekannt. In letzte Zeit sind verstärkt Puder mit höheren Wasseranteilen beschrieben worden. Die US-B-6290941 offenbart Puder/Flüsigkeits-Zusammensetzungen, die hydrophob beschichtetes Siliciumdioxid mit Wasser und einem wasserlöslichen Polymeren enthalten und dabei ölfrei sind.

EP 1386599 A1 offenbart ein Verfahren zur Massenproduktion von "trockenem Wasser", wobei die Puderzusammensetzungen 1,3-Butylenglycol als Feuchthaltemittel enthalten.

Der Erfindung liegt die Aufgabe zugrunde, einen kosmetischen Puder zu entwickeln, der bei sehr hohen Wasseranteilen stabil formuliert und rieselfähig ist und zugleich eine verbesserte UV-Stabilität ohne Zusatz von UV-Filtern aufweist.

Erfindungsgemäß besteht der kosmetische Puder auf Siliciumdioxid-Basis aus 1-9 Gew-% hydrophobem, feinteiligem Siliciumdioxid mit einer BET-Oberfläche* im Bereich von 180-280 m²/g und einer mittleren Teilchengröße von 5-30 nm,
20-95 Gew-% Wasser und 0,1-20 Gew-% Sorbitol als Feuchthaltemittel, wobei das Verhältnis SiO₂ zu Wasser im Bereich von 1:11 bis 1:30 liegt und die Prozentangaben auf das Gesamtgewicht des rieselfähigen Puders bezogen sind,
und wobei der Puder kein Öl, keine wasserlöslichen PVP-, VA-, Acryl- oder Urethan-Polymere oder Hydrokolloide enthält.
* (bestimmt gemäß J. Amer.Chem.Soc. **60**(1938)309 ff).

Der erfindungsgemäße Puder kann sehr hohe Wasseranteile aufweisen und dennoch zu eine stabilen Puderformulierung bilden, in der der Puder rieselfähig bleibt. Darüber hinaus zeigt ein solcher Puder eine sehr hohe UV-Stabilität, und die Abscheidung von Wasser am Behälterboden erfolgt auch bei längerer direkter Sonneneinstrahlung nicht. Dies ermöglicht die Herstellung eines stabileren Endproduktes, das auch eine Vermischung mit anderen pulverförmigen Bestandteilen zuläßt, wie Cyclodextrinkapseln oder KSP 100 (Dimethicone/Vinyl Dimethicone Crosspolymer). Der Puder enthält kein Öl und auch keine wasserlöslichen PVP-, VA-, Acryl- oder Urethan-Polymere wie in der US-B-6290941, mit Ausnahme von Parfümölen oder aromatischen Rohmaterialien. Er enthält auch keine hydrophilen Kolloide, die netzartige Strukturen ausbilden können.

Das Verhältnis SiO₂ zu Wasser liegt vorzugsweise im Bereich von 1:15 bis 1:25, insbesondere 1:11 bis 1:19.

Bevorzugte Sorbitolanteile liegen im Bereich von 0,1 bis 10 Gew-%.

Bevorzugte Wasseranteile liegen im Bereich von 40-95 Gew-%, vorteilhaft 60-95 Gew-%, insbesondere 72-95 Gew-%, speziell 82-95 Gew-%.

Der Gehalt an hydrophobem, feinteiligem Siliciumdioxid liegt vorteilhaft im Bereich von 1 bis 7 Gew-%. Das Siliciumdioxid ist vorzugsweise ein mit einem Silan-amin hydrophobiertes Silica mit einem Kohlenstoffanteil von 3-4 Gew-% und dessen Teilchengröße im Bereich von 5-12 nm liegt.

Der erfindungsgemäße kosmetische Puder kann weitere kosmetische Bestandteile enthalten, wie Pflanzenextrakte, Hefeextrakte, Vitamine, Panthenol, beschichtete und unbeschichtete Pigmente wie Glitzer- und Perlglanzpigmente, andere pulverförmige Bestandteile wie Bentonit oder Bornitrid, verkapselte oder unverkapselte Parfümöle wie z.B in Cyclodextrinen verkapselte Parfümöle, Konservierungsmittel, Schutzmittel, Dimethicone/Vinyl Dimethicone Crosspolymer, organische und anorganische UV-Filter wie Zimtsäure- und Benzophenonderivate sowie TiO₂ und ZnO und Gemische davon.

Als Hefeextrakte können z.B. wenigstens 0,5 Gew-% eines Gemisches aus Enzymen und Vitaminen, das wenigstens 150 Einheiten/ml (U/ml) Superoxiddismutase enthält, eingesetzt werden, hergestellt gemäß DE 4241154 C1. Ein solches Gemisch aus Enzymen und Vitaminen ist vorzugsweise ein durch Ultraschallbehandlung hergestelltes Aufschlußprodukt einer Hefe, wobei das Aufschlußprodukt SOD, Protease, Vitamin B₂, Vitamin B₆, Vitamin B₁₂, Vitamin D₂ und Vitamin E enthält. Vorzugsweise enthält es wenigstens 150 U/ml SOD, Protease und die Vitamine B und D, wobei das Verhältnis SOD:Protease als internationale Einheiten wenigstens im Bereich von 3:1 bis 8:1 liegt.

Als Pflanzenextrakte können 0,01 bis 1 Gew-% einer komplexen Wirkstoffzubereitung mit hohem Radikalschutzfaktor (RPF) mit einem Gehalt an einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und weiterhin Phospholipide und Wasser. Ebenso in der komplexen Wirkstoffzubereitung enthalten sein können das durch Ultraschallbehandlung hergestellte Aufschlußprodukt einer Hefe, das wenigstens 150 Einheiten Superoxiddismutase pro ml enthält und ein oder mehrere Cyclodextrine. Die komplexe Wirkstoffzubereitung kann ein Produkt gemäß W099/66881 Beispiel 1 oder 2 oder WO 01/26617 (002467) Beispiel 1 sein.

Weitere Pflanzenextrakte sind vollständig klare alkoholische Extrakte aus roten Äpfeln, grünen Äpfeln oder ein Extraktgemisch aus roten und grünen Äpfeln, wobei dies ein Extrakt der Gesamtfrucht ist, oder ein kosmetischer Basiskomplex, bestehend aus einem verkapselten Extrakt einer wäßrigen Extraktion der Ananas-Frucht und dem Rückstand einer wäßrigen Extraktion von Joghurt.

Andere mögliche Bestandteile sind modifizierte kupferbindende Proteine, denen das Kupfer in der prosthetischen Gruppe durch eine vorherige Austauschreaktion entzogen wurde oder die durch rekombinante Verfahren ohne Kupfer hergestellt wurden und die Hautaufhellungsmittel sind. Beispiele dafür sind Azurin, Ascorbatoxidase, Laccase, Auracyanin usw. Weitere Bestandteile können gemahlene, transparente Glasteilchen mit einer mittleren Korngröße von 10 bis 30 nm und einem Anteil von 0,1 bis 80 Gew-% sein, wobei die Glasteilchen aus einem geschmolzenen und danach erstarrten Glas gebildet werden. Bevorzugte Glasteilchen sind Glasteilchen von Infrarötgläsern mit einer Teilchengröße von 10-30 nm.

Vitamine und andere Radikalfänger können ebenfalls zugesetzt werden, wie Vitamin C und Derivate davon, beispielsweise Ascorbylacetat, -phosphat und -palmitat; Folsäure und deren Derivate; wasserlösliches Vitamin E und dessen Derivate, wie Tocopherylacetat; Flavone oder Flavonoide; Aminosäuren, wie Histidin, Glycin, Tyrosin, Tryptophan und Derivate davon; Carotinoide und Carotine, wie z.B alpha-Carotin, β-Carotin; Harnsäure und Derivate davon; alpha-Hydroxysäuren wie Citronensäure, Milchsäure, Äpfelsäure. Sofern diese Substanzen öllöslich sind, ist eine Absorption oder Verkapselung zur Umwandlung in eine Pulverform erforderlich.

Parfümkomponenten oder aromatische Rohmaterialien sind meist öllösliche Produkte. Sie können ebenfalls im breiten Maße eingesetzt werden, indem sie nach Absorption pulverförmig mit dem Emulsionspuder vermischt werden. Es können alle klassischen technischen Wege zur Überführung von Duftstoffen in Puderform beschritten werden. Sie können absorbiert/ adsorbiert oder verkapselt werden mit solchen Stoffen wie Cyclodextrinen, Silica, Bentonit, Zeolith, oder Stearinsäure als mikronisierte Pulver oder Wachsverbindungen, Aromatische Rohmaterialien sind wasserlöslich oder wassermischbar. Sie können direkt in der Wasserphase eingesetzt werden. Beispiele dafür sind Phenoxyethylalkohol, Phenylethylalkohol, Benzylalkohol, Methylpyrazin, Anisylalkohol, Linaloloxid, Maltol, Calone, Kresol, Dimethylbenzylcarbinol, Vanillin, Ethylacetylacetat, Ethylmaltol, Hexenol-3-cis oder Ethylacetat. Ebenfalls möglich sind einige florale Wässer als Parfüm.

Gegenstand der Erfindung ist auch ein Verfahren zur Herstellung des kosmetischen Puders. Dabei vermischt man ein erstes Gemisch, das wenigstens Wasser und Sorbitol enthält, mit einem hydrophoben, feinteiligen Siliciumdioxid mit einer BET-Oberfläche im Bereich von 180-280 m²/g und einer mittleren Teilchengröße von 5-30 nm im Verhältnis SiO₂:H₂O im Bereich von 1:11 bis 1:30 mit einem mechnischen Mischer und 1000 bis 3000 U/min für einen Zeitraum von 3 bis 15 Minuten zu einem zweiten Gemisch. Weitere kosmetische Zusatzstoffe können entweder dem Wasser vor der Emulgierung mit dem Siliciumdioxid (flüssige) oder dem zweiten Pudergemisch aus Siliciumdioxid und Wasser (puderförmige) zugesetzt werden.

Der Mischvorgang wird vorzugsweise für einen Zeitraum von 4 bis 9 Minuten durchführt, beispielsweise mit einem vierblättrigen Propellerrührer bei etwa 1500 U/min.

Der erfindungsgemäße Puder führt zu einem sehr angenehmen Frischegefühl auf der Haut. Er ist UV-stabil und ist ein Verstärker (booster) für gegebenenfalls darin enthaltene UV-Filter.

Die Erfindung soll nachstehend durch Beispiele näher erläutert werden. Alle Angaben erfolgen in Gewichtsprozent, sofern nichts anderes angegeben ist.

### Beispiel 1 Glimmer-Wasserpuder nicht erfindungsgemäß

| **Phase A** | |
|---|---|
| Wasser | 17,9 |
| Sorbitol | 1 |
| Konservierungsmittel | 0,1 |

| **Phase B** | |
|---|---|
| Sylitate silica^{*} | 1 |

| **Phase C** | |
|---|---|
| Mica | 80 |
| Ascorbinsäure-Pulver | 0,1 |

| | |
|---|---|
| ^{*} Siliciumdioxid BET-Oberfläche 190-250 m?/g, C-Gehalt 3-4 %, mittlere Teilchengröße 7 nm. | |

Die Phase A wurde hergestellt durch Vermischen des Schutzmittels und des Feuchthaltemittels nacheinander mit dem Wasser unter Rühren mit einem RAYNERI TURBOTEST 33/750P mit einem 80 mm Durchmesser Propeller bei etwa 3000 U/min für 1 Minute. Danach wurde das Siliciumdioxid zugegeben und für 10 Minuten bei etwa 1000 U/min gerührt. Die Phase C wurde zum Schluß unter Rühren zugegeben (ca. 1 Minute).

### Beispiel 2 Parfüm-Wasserpuder

| **Phase A** | |
|---|---|
| Wasser | 89,5 |
| Sorbitol | 5 |
| Konservierungsmittel | 0,5 |

| **Phase B** | |
|---|---|
| Sylitate silica | 5 |

Die Herstellung erfolgte wie im Beispiel 1.

### Beispiel 3 Vitamin-Wasserpuder

| **Phase A** | |
|---|---|
| Wasser | 72,6 |
| Sorbitol | 20 |
| Konservierungsmittel | 0,4 |
| Panthenol D-50 | 2 |

| **Phase B** | |
|---|---|
| Sylitate silica | 5 |

### Beispiel 4Pflanzenextrakt-Wasserpuder

| **Phase A** | |
|---|---|
| Wasser | 94,3 |
| Sorbitol | 0,1 |
| Konservierungsmittel | 0,5 |
| Hefeextrakt | 0,1-0,5 |

| **Phase B** | |
|---|---|
| Sylitate silica | 5 |

Die Herstellung erfolgte wie im Beispiel 1 ohne die Phase C.

### Beispiel 5 Schwachfarbiger Wasserpuder

| **Phase A** | |
|---|---|
| Wasser | 86 |
| Sorbitol | 5 |
| Konservierungsmittel | 0,5 |
| Farbstoff Rot | 0,08 |
| Farbstoff Gelb | 0,048 |
| Farbstoff Grün (0,1 % in Wasser) | 3,372 |

| **Phase B** | |
|---|---|
| Sylitate silica* | 5 |

Die Herstellung erfolgte wie im Beispiel 1 ohne die Phase C.

### Beispiel 7 Parfüm-Wasserpuder II

| **Phase A** | |
|---|---|
| Wasser | 83,5 |
| Sorbitol | 5 |
| Konservierungsmittel | 0,5 |

| **Phase B** | |
|---|---|
| Sylitate silica | 5 |

| **Phase C** | |
|---|---|
| Parfümöl | 3 |
| Methylmethacrylate Crosspolymer | 3 |

Die Herstellung erfolgte wie im Beispiel 1, wobei die Phase C durch separates Vermischen der beiden Bestandteile in einem Pulvermischer bis zur vollständigen Adsorption des Parfümöls an das PMMA-Pulver erfolgte.

### Beispiel 6 Vergleichsbeispiel

Die vorteilhaften Eigenschaften des erfindungsgemäßen Puders in Bezug auf die UV-Stabilität wurden durch Vergleichsversuche bestätigt.

Eine Zusammensetzung gemäß Beispiel 2 (Probe A) wurde in einem SUNTEST-Simulator über einen Zeitraum von 12 Stunden mit kurzwelligem UV-Licht (296 nm bis 400 nm) und durch ein 12 im Glasfilter bestrahlt. Die gleiche Behandlung erfolgte mit einer Zusammensetzung, die 0% Sorbitol enthielt (Probe B) und die 7% Sorbitol enthielt (Probe C). Die Ergebnisse sind in Tabelle 1 aufgeführt.

**Tabelle 1**

| UV- Bestrahlung | | | |
|---|---|---|---|
| | nach 4 Std. | nach 8 Std. | nach 12 Std. |
| Probe A | X | X | □ |
| Probe B | □ | O | O |
| Probe C | X | X | X |

| | | | |
|---|---|---|---|
| X = vollständig stabile Emulsion □ = geringe Wasserabscheidung am Behälterboden O = starke Wasserabscheidung am Behälterboden | | | |

## Patentansprüche

1. Kosmetischer Puder auf Siliciumdioxid-Basis,
**gekennzeichnet durch** einen Gehalt an
1-9 Gew-% hydrophobem, feinteiligem Siliciumdioxid mit einer BET-Oberfläche im Bereich von 180-280 m²/g und einer mittleren Teilchengröße von 5-30 nm,
20-95 Gew-% Wasser und
0,1-20 Gew-% Sorbitol, wobei das Verhältnis SiO₂ zu Wasser im Bereich von 1:11 bis 1:30 liegt und alle Prozentangaben auf das Gesamtgewicht des rieselfähigen Puders bezogen sind, und wobei der Puder kein Öl, mit Ausnahme von Parfümölen keine wasserlöslichen PVP-, VA-, Acryl- oder Urethan-Polymere oder Hydrokolloide enthält.

2. Puder nach Anspruch 1, **dadurch gekennzeichnet, dass** das Verhältnis SiO₂ zu Wasser im Bereich von 1:11 bis 1:25 liegt.

3. Puder nach Anspruch 1, **dadurch gekennzeichnet, dass** der Gehalt an hydrophobem, feinteiligem Siliciumdioxid im Bereich von 1 bis 7 Gew-% liegt.

4. Puder nach Anspruch 1, **dadurch gekennzeichnet, dass** er weitere kosmetische Bestandteile enthält, ausgewählt unter Pflanzenextrakten, Vitaminen, Panthenol, beschichteten und unbeschichteten Pigmenten, Glaspulvern, modifizierten kupferbindenden Proteinen, Farbstoffen, floralen Wässern, wasserlöslichen aromatischen Rohmaterialien, wassermischbaren aromatischen Rohmaterialien und Gemischen davon.

5. Puder nach Anspruch 1, **dadurch gekennzeichnet, dass** der Wasseranteil im Bereich von 72-95 Gew-% liegt, vorzugsweise 82-95 Gew-%.

6. Verfahren zur Herstellung eines kosmetischen Puders nach Anspruch 1, **dadurch gekennzeichnet, dass** man ein Gemisch, das wenigstens Wasser und Sorbitol enthält, mit einem hydrophoben, feinteiligen Siliciumdioxid mit einer BET-Oberfläche im Bereich von 180-280 m²/g und einer mittleren Teilchengröße von 5-30 nm im Verhältnis SiO₂:H₂O im Bereich von 1:11 bis 1:30 mit einem mechanischen Mischer und 1000 bis 3000 U/min für einen Zeitraum von 3 bis 15 Minuten vermischt, und weitere flüssige Bestandteile dem Wasser vor der Emulgierung mit Siliciumdioxid zufügt oder weitere puderförmige Bestandteile dem Pudergemisch aus Siliciumdioxid und Wasser zufügt.

7. Verfahren nach Anspruch 6, **dadurch gekennzeichnet, dass** man den Mischvorgang für einen Zeitraum von 4 bis 9 Minuten durchführt.

8. Verfahren nach Anspruch 7, **dadurch gekennzeichnet, dass** man einen Duftstoff als in eine puderförmige Form überführte Parfümkomponente oder als aromatisches Rohmaterial zusetzt.

## Claims

1. A cosmetic powder on a silica base, **characterised by** a content of
1-9 % by weight hydrophobic, fine-structured silica with a BET surface in the range of 180-280 m²/g and a mean particle size of 5 - 30 nm,
20-95 % by weight of water, and
0.1-20 % by weight of sorbitol,
with the SiO₂ : water ratio being in the range of 1 : 11 to 1 : 30 and all percentages relating to the total weight of the pourable powder,
and wherein the powder does not contain any oil, watersoluble PVP, VA, acrylic or urethane polymers or hydro colloids, with exception of perfume oils.

2. Powder according to claim 1, **characterized in that** the SiO₂: water ratio is in the range of 1 : 11 to 1 : 25.

3. Powder according to claim 1, **characterized in that** the content of hydrophobic, fine-structured silicon dioxide is in the range of 1 to 7 % by weight.

4. Powder according to claim 1, **characterized in that** it contains further cosmetic ingredients, selected from plant extracts, vitamins, panthenol, coated and uncoated pigments, glass powders, modified copper-binding proteins, dyes, floral waters, water soluble aromatic raw materials, water miscible aromatic raw materials, and mixtures thereof.

5. Powder according to claim 1, **characterized in that** the water percentage is in the range of 72-95 % by weight, preferably 82-95 % by weight.

6. A method for the preparation of a cosmetic powder according to claim 1, **characterized in that** a mixture containing at least water and sorbitol is mixed with a hydrophobic, fine-structured silica with a BET surface in the range of 180-280 m²/g and a mean particle size of 5-30 nm in the ratio SiO₂ : H₂O in the range of 1 : 11 to 1 : 30 by means of a mechanical mixer and 1000 to 3000 rpm for a period of 3 to 15 minutes and that further liquid ingredients are added in water before emulsion with silica or further powder ingredients are added to the powder mixture of silica and water.

7. Method according to claim 6, **characterized in that** the mixing is carried out over a period of 4 to 9 minutes.

8. Method according to claim 7, **characterized in that** a fragrance is added as powder transformed perfume compound or as aromatic raw material.

## Revendications

1. Poudre cosmétique à base de dioxyde de silicium, **caractérisée par** une teneur de 1 à 9 % en poids de dioxyde de silicium en fine particules, hydrophobe, ayant une surface spécifique BET dans une gamme de 180 à 280 m²/g et une granulométrie moyenne de 5 à 30 nm,
20 à 95 % d'eau et
0,1 à 20 % en poids de sorbitol, moyennant quoi le rapport de SiO₂ à l'eau est dans une gamme de 1 : 11 à 1 : 30 et toutes les indications en pourcentage se rapportent au poids total de la poudre coulante, et moyennant quoi la poudre ne contient pas d'huile, à l'exception des huiles de parfum, ni de polymères hydrosolubles de PVP, VA, d'acryle ou d'uréthane, ni d'hydrocolloïdes.

2. Poudre selon la revendication 1, **caractérisée en ce que** le rapport du SiO₂ à l'eau est dans une gamme de 1 : 10 à 1 : 25.

3. Poudre selon la revendication 1, **caractérisée en ce que** la teneur en dioxyde de silicium en fine particules, hydrophobe est dans une gamme de 1 à 7 % en poids.

4. Poudre selon la revendication 1, **caractérisée en ce qu'**elle contient d'autres constituants cosmétiques, choisis parmi les extraits végétaux, les vitamines, le panthénol, les pigments recouverts ou non recouverts, les poudres de verre, les protéines modifiées liant le cuivre, les colorants, les eaux florales, les matières premières aromatiques hydrosolubles, les matières premières aromatiques miscibles à l'eau et les mélanges d'entre eux.

5. Poudre selon la revendication 1, **caractérisée en ce que** la proportion d'eau est dans une gamme de 72 à 95 % en poids, de préférence de 82 à 95 % en poids.

6. Procédé de préparation d'une poudre cosmétique selon la revendication 1, **caractérisé en ce qu'**on agite un mélange qui contient au moins de l'eau et du sorbitol avec un dioxyde de silicium à fines particules, hydrophobe, ayant une surface spécifique BET dans une gamme de 180 à 280 m²/g et une granulométrie moyenne de 5 à 30 nm, dans un rapport SiO₂/H₂O dans une gamme de 1 : 11 à 1 : 30, avec un mélangeur mécanique, à une vitesse de 1000 à 3000 t/min, pendant une durée de 3 à 15 minutes, et on ajoute d'autres constituants liquides à l'eau avant l'émulsification avec le dioxyde de silicium ou on ajoute d'autres constituants poudreux au mélange poudreux de dioxyde de silicium et d'eau.

7. Procédé selon la revendication 6, **caractérisé en ce qu'**on effectue l'opération de mélange pendant une durée de 4 à 9 minutes.

8. Procédé selon la revendication 7, **caractérisé en ce qu'**on ajoute un composant odoriférant à titre de composante de parfum réduit sous forme poudreuse ou à titre de matière première aromatique.
